# EUROPÄISCHE PATENTANMELDUNG

(11) **EP 1 174 422 A2**
(43) Veröffentlichungstag der Anmeldung: **23.01.2002**
(21) Anmeldenummer: 01116476.1
(22) Anmeldetag: 06.07.2001
(51) Int. Cl.: C07C 319/14, C07C 231/10, C07C 321/28

(54) **Umweltfreundliches Verfahren zur Herstellung sterisch gehinderter Phenole mit höherem Molekulargewicht**

(30) Priorität: 21.07.2000 DE 10036480
(71) Anmelder: Institut für Polymerforschung Dresden e.V., 01069 Dresden (DE)
(72) Erfinder: Böhme, Frank, Dr., 01705 Pesterwitz (DE); Luston, Jozef, Dr., 81109 Brastilava (SK)
(74) Vertreter: Rauschenbach, Marion, Dipl.-Ing.

(57) **Zusammenfassung**

Die Erfindung bezieht sich auf das Gebiet der Chemie und betrifft ein umweltfreundliches Verfahren zur Herstellung sterisch gehinderter Phenole mit höherem Molekulargewicht, die beispielsweise zur thermischen Stabilisierung von Polymeren eingesetzt werden können.

Der Erfindung liegt die Aufgabe zugrunde, sterisch gehinderte Phenole anzugeben, die thermisch und/oder thermooxidativ stabilisierend auf ein Polymer wirken.

Gelöst wird die Aufgabe durch ein umweltfreundliches Verfahren zur Herstellung sterisch gehinderter Phenole mit höherem Molekulargewicht der allgemeinen Formel (I), 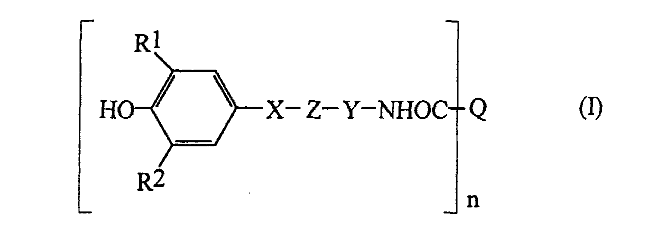 bei dem eine Verbindung der Formel (III) mit einer Verbindung der Formel (IV) 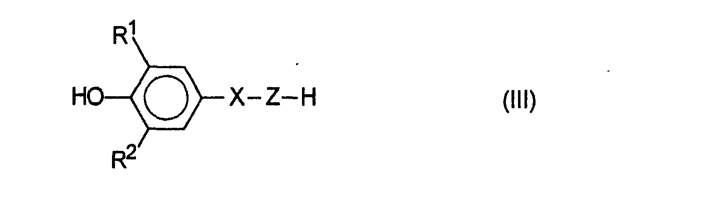 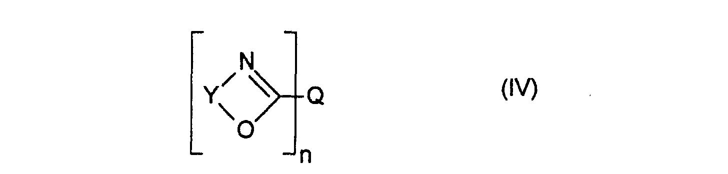 zur Reaktion gebracht wird.

## Beschreibung

Die Erfindung bezieht sich auf das Gebiet der Chemie und betrifft ein umweltfreundliches Verfahren zur Herstellung sterisch gehinderter Phenole mit höherem Molekulargewicht, die beispielsweise zur thermischen Stabilisierung von Polymeren eingesetzt werden können.

Es ist bekannt, daß sterisch gehinderte Phenole eine sehr gute Wirkung bezüglich des thermischen und thermo-oxidativen Abbaus verschiedener Polymere zeigen (J. Pospí 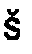 il, Antioxidanty, Academia Prag, 1968). Sie inhibieren radikale Prozesse in Polymeren und verlängern dadurch deren Lebensdauer. Es ist ebenfalls gut bekannt, daß niedermolekulare Additive häufig durch Verdampfen, Sublimation und Extraktion aus dem Polymer entweichen (J. Luston, in Developements in Polymer Stabilization-2, Ed. G. Scott, Applied science Publishers, London 1980, Chap. 5; N. C. Billingham, P. D. Calvert in Developements in Polymer Stabilization - 3. Ed. G. Scott, Applied Science Publishers, London 1980, Chap. 5, N. C. Billingham in Oxidation Inhibition in Organic Materials, Vol. 2, Eds J. Pospí 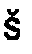 il, P. P. Klemchuk, CRC Press, Boca Raton, 1990, p. 249 ). Durch den physikalischen Verlust der Additive verringert sich die Lebensdauer der Polymere. Aus diesem Grund werden Stabilisatoren mit hohem Molekulargewicht oder Stabilisatoren, die direkt an die Polymerkette angebunden sind, hergestellt und in Polymersystemen angewendet (J. A. Kuczkowski, J. G. Gillick in Rubber Chem. Technol. 57, 621 (1984), J. Pospí 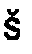 il in Oxidation Inhibition in Organic Materials, Vol. 1, Eds J. Pospí 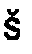 il, P. P. Klemchuk, CRC Press, Boca Raton, 1990, p. 193; J. Pospí 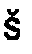 il in Adv. in Polymer Science 101 Springer Verlag, Berlin 1991, p. 65). Ein anderer Vorteil von Stabilisatoren mit hohem Molekulargewicht besteht darin, daß ihre Toxizität gewöhnlich viel geringer als die von niedermolekularen Stabilisatoren ist. Das ist auf ihre geringere Löslichkeit in normalen Lösungsmitteln, einschließlich Wasser, zurückzuführen. Kürzlich wurde auch über einige Verfahren berichtet, welche die Synthese polymerer bzw. reaktiver Antioxidantien betrifft (D. Munteanu, C. Csunderlik, I. Tincul; J. Thermal Anal. 37 411 (1991); D. Munteanu, C. Csunderlik, Polym. Degrad. Stabil. 34. 295 (1991), J. M. Herdan, M. Stan, M. Giurginca, Polym. Degrad. Stabil. 50. 59 (1995); M. A. Tlenkopatchiev, E. Miranda, R. Gabino, T. Ogawa, Polym. Bull. 35, 547 (1995)).

Es existieren verschiedene Möglichkeiten das Molekulargewicht aktiver Spezies zu erhöhen. Wie ober erwähnt, kann das über die Funktionalisierung der aktiven Spezies und deren nachfolgende Polymerisation oder Copolymerisation mit nichtaktiven Monomeren, durch Pfropfung der aktiven Spezies an ein Polymer und/oder durch Kopplung mehrerer niedermolekularer funktioneller Verbindungen erfolgen. Um die Flüchtigkeit von Polymerstabilisatoren zu vermindern, werden Molekulargewichte größer als 500 empfohlen (J. Luston, in Developments in Polymerstabilisation - 2, Ed. G. Scott, Applied Science Publishers, London 1980, Chapter 5).
Höhermolekulare sterisch gehinderte Phenole mit Ester- und Esteramidgruppierungen wurden bereits in der Literatur beschrieben. In der DE 37 02 755 A1 werden verschiedene Derivate des 3,5-Di-tert-butyl-4-hydroxybenzamids und deren Einsatz für therapeutische Zwecke beschrieben. Sterisch gehinderte Phenole mit zusätzlichen Aminoamidstrukturen werden in der DE 35 21 558 A1 beschrieben. In der US 4 066 610 wird auf die stabilisierende Wirkung von Amino- und Amidostrukturen in Bezug auf komplexbildende Eigenschaften von sterisch gehinderten Phenolen hingewiesen. Amidoesterstrukturen in Verbindungen sterisch gehinderter Phenolen werden in der US 3 992 434 beschrieben. Zimtsäureamidderivate mit zusätzlichen Amino-, Amido- und/oder Esterbindungen sowie einer Anzahl weiterer Strukturelemente mit antihyperlipidemischer Aktivität sind im EP 0 407 200 A1 angegeben. Verbindungen mit zwei über verschiedene Amido-, Thioamido-, Ester-, Thioester-, Harnstoff- und/oder Thioharnstoffgruppierungen verknüpften sterisch gehinderten Phenolgruppen sind durch das EP 0 404 039 als antiarteriosklerotisches Agenz bekannt. Vinylphosphonsäureamide mit weiteren Amido- und 3,5-Di-tert-butyl-4-hydroxyphenylgruppen im Molekül sind Gegenstand der SU 644 795. Eine Amidoesterphthalatgruppierung als Verknüpfung zweier sterisch gehinderter Phenole ist aus der SU 567 719 bekannt.

Jede dieser Strukturen wurde durch Kondensation des entsprechenden Säureäquivalents mit einem Amin, Alkohol oder einer halogenierten Verbindung hergestellt. In einigen Beispielen wird als Ausgangsverbindung das Chlorid einer 3,5-Di-tert-butyl-4-hydroxyphenyl-Einheit enthaltenden Säure, welche durch die Reaktion mit einem Amin, Diamin oder Alkohol die gewünschte Verbindung ergibt, eingesetzt (DE 37 02 755 A1, US 4 066 610, US 3 992 434, EP 04 07 200 A1). Verbindungen mit zwei über eine Amido- oder Amidoestergruppierung verknüpften sterisch gehinderten Phenolgruppen können ebenfalls aus Vinylphosphonsäuredichloriden oder Dichloriden der Phthal- oder Terephthalsäure über Kondensationsreaktionen mit aminoamid- oder hydroxyamidenthaltenden Derivaten sterisch gehinderter Phenole (SU 644 795, SU 567 719) hergestellt werden. Die direkte Kondensation von Säuren, welche die 3,5-Di-tert-butyl-4-hydroxyphenyl-Einheit enthalten, mit Aminen (DE 37 02 755 A1, US 3 992 434, EP 04 07 200 A1, EP 04 04 039 A1) in Gegenwart einer Base oder eines dehydratisierenden Agenz (Dizyklohexylcarbodiimid oder 1-Ethyl-3-(3-dimethylaminopropyl)carbodiimid) ist ebenfalls möglich.

Es ist offensichtlich, dass in allen oben genannten Fällen Nebenprodukte wie Hydrogenchlorid, seine Amoniumsalze, Harnstoffderivate etc. gebildet werden, deren Beseitigung Umweltprobleme verursachen und zu hohen Preisen des Endprodukts führen.

Aus dem DE 197 47 644 A1 ist weiterhin die Synthese und Anwendung hochmolekularer Antioxidantien basierend auf sterisch gehinderten Phenolen mit geringer Flüchtigkeit bekannt. Die Verbindungen werden durch Umsetzung von 2-oxazolingruppenenthaltenden Derivaten sterisch gehinderter Phenole mit diversen substituierten oder unsubstituierten, mono- oder polyfunktionellen, aliphatischen, zykloaliphatischen, aromatischen und heterozyklischen Carbonsäuren, Thiolen und Phenolen erhalten. Alle auf diese Weise synthetisierten Verbindungen enthalten Amidgruppierungen, welche zusätzliche Wechselwirkungen mit dem Polymer eingehen können. Obwohl diese Methode die Herstellung zahlreicher hochmolekularer Antioxidantien ohne die Bildung von Nebenprodukten ermöglicht, besteht ein Nachteil darin, dass es mit diesem Verfahren nicht möglich ist, Antioxidantien mit zusätzlich komplexierenden Eigenschaften zu erhalten.
Ein weiterer Nachteil besteht in der aufwendigen Synthese der mit 2-Oxazolingruppen funktionalisierten sterisch gehinderten Phenole.

Der Erfindung liegt die Aufgabe zu Grunde, ein von Nebenprodukten weitgehend freies Verfahren zur Herstellung sterisch gehinderter Phenole mit höherem Molekulargewicht, welche thermisch und/oder thermooxidativ stabilisierend auf ein Polymer wirken, in der Lage sind Katalysatoren zu komplexieren und im geringem Maße aus dieser stabilisierten Verbindung entweichen, anzugeben, bei dem weitestgehend auf leicht verfügbare und/oder leicht herstellbare Ausgangsverbindungen zurückgegriffen werden kann.

Bei dem erfindungsgemäßen Verfahren erfolgt die Umsetzung von Verbindungen mit einer oder mehreren 2-Oxazolingruppen mit Derivaten sterisch gehinderter Phenole, welche reaktive Carboxyl- oder Thiolgruppen enthalten.

Bei dem erfindungsgemäßen Verfahren erfolgt die Synthese von Verbindungen, mit einer oder mehreren 3,5-Dialkyl-4-hydroxyphenyl-Gruppen im Molekül entsprechend der Formel (I), 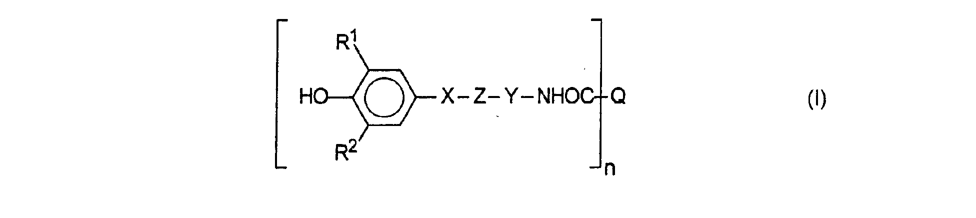
wobei R¹ und R² gleiche oder unterschiedliche, lineare oder verzweigte, monovalente Kohlenwasserstoffgruppen mit 1 bis 8 Kohlenstoffatomen,
X eine einfache chemische Bindung oder eine gesättigte oder ungesättigte Kohlenwasserstoffgruppe mit 1 oder 2 Kohlenstoffatomen,
Z eine -S- oder -COO-Gruppe,
Y eine Kohlenwasserstoffgruppe mit 2 oder 3 Kohlenstoffatomen,
Q ein mono- oder polyvalentes Radikal bestehend aus einer aliphatischen oder zykloaliphatischen gesättigten oder ungesättigten, linearen oder verzweigten Kohlenwasserstoffgruppe mit 1 bis 20 Kohlenstoffatomen, oder
ein mono- oder polyvalentes Radikal bestehend aus einer aliphatischen oder zykloaliphatischen, gesättigten oder ungesättigten, linearen oder verzweigten Kohlenwasserstoffgruppe mit 1 bis 20 Kohlenstoffatomen und mindestens einem der Heteroatome S, O, P oder N, oder
ein mono- oder polyvalentes nichtsubstituiertes oder substituiertes aromatisches Radikal bestehend aus 6 bis 20 Kohlenstoffatomen, oder
ein mono- oder polyvalentes nichtsubstituiertes oder substituiertes heteroaromatisches Radikal bestehend aus 5 bis 20 Kohlenstoffatomen, oder
ein mono- oder polyvalentes nichtsubstituiertes oder substituiertes Aralkylradikal bestehend aus 6 bis 20 Kohlenstoffatomen, oder
eine Biarylverbindung der allgemeinen Formel (II),
wobei A entweder eine chemische Bindung, eine Methylen-, Isopropyliden-, Isobutyliden-, -S-, -SO-, -SO₂-, -O-, -C(O)-, -OC(O)O- oder -C(O)O-Gruppe repräsentieren, und
n eine ganze Zahl von 1 bis 6 ist.

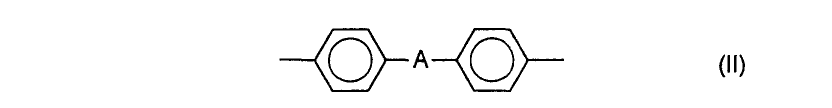

In der Verbindung der Formel (I) sind R¹ und R² bevorzugt tertiäre Butylgruppen und X ist vorzugsweise eine chemische Bindung oder eine Kohlenwasserstoffkette mit zwei Kohlenstoffatomen, indem eine Verbindung nach Formel (III) mit einer Verbindung der allgemeinen Formel (IV) zur Reaktion gebracht wird, vorzugsweise in Schmelze oder in Lösung eines oder mehrerer hochsiedender Lösungsmittel, 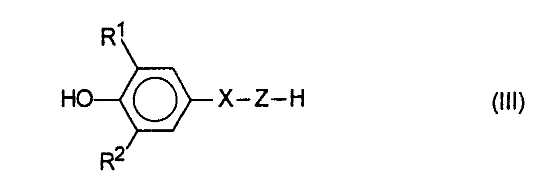 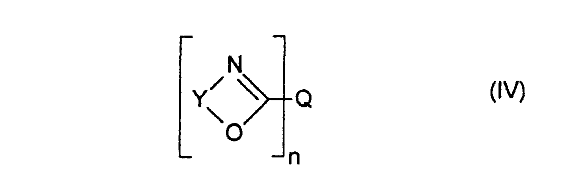
wobei in der Verbindung nach Formel (III) R¹ und R² gleiche oder unterschiedliche lineare oder verzweigte monovalente Kohlenwasserstoffgruppen mit 1 bis 8 Kohlenstoffatomen,
X eine einfache chemische Bindung oder eine gesättigte oder ungesättigte Kohlenwasserstoffgruppe mit 1 bis 2 Kohlenstoffatomen, und
Z eine -S- oder -COO-Gruppe ist, und
in der Verbindung (IV)
Y eine Kohlenwasserstoffgruppe mit 2 oder 3 Kohlenstoffatomen, Q ist ein mono- oder polyvalentes Radikal bestehend aus einer aliphatischen oder zykloaliphatischen · gesättigten oder ungesättigten, linearen oder verzweigten Kohlenstoffgruppe mit 1 bis 20 Kohlenstoffatomen, oder
ein mono- oder polyvalentes Radikal bestehend aus einer aliphatischen oder zykloalipathischen, gesättigten oder ungesättigten, linearen oder verzweigten Kohlenwasserstoffgruppe mit 1 bis 20 Kohlenstoffatomen und mindestens einem der Heteroatome S, O, P oder N, oder
ein mono- oder polyvalentes nichtsubstituiertes oder substituiertes aromatisches Radikal bestehend aus 6 bis 20 Kohlenstoffatomen, oder
ein mono- oder polyvalentes nichtsubstituiertes oder substituiertes heteroaromatisches Radikal bestehend aus 5 bis 20 Kohlenstoffatomen, oder
ein mono- oder polyvalentes nichtsubstituiertes oder substituiertes Aralylradikal bestehend aus 6 bis 20 Kohlenstoffatomen, oder
eine Biarylverbindung der allgemeinen Formel (II),
wobei A eine chemische Bindung, eine Methylen-, eine Isopropylen-, eine Isobutylen, eine -S-, -SO-, -SO₂-, -O-, -C(O)-, -OC(O)O- oder -C(O)O-Gruppe repräsentiert, und
n eine gerade Zahl von 1 bis 6 ist.

Bei der Reaktion in Schmelze erfolgt die Herstellung in einem Temperaturbereich oberhalb des Schmelzpunktes von mindestens einer der Reaktionskomponenten, vorzugsweise im Temperaturbereich zwischen 100 und 260 °C, noch bevorzugter im Temperaturbereich zwischen 130 und 200 °C, in inerter Atmosphäre während einer Zeit von 1 Minute bis 6 Stunden, vorzugsweise während einer Zeit von 3 Minuten bis 1 Stunde je nach Reaktionstemperatur, Schmelzpunkt der reagierenden Komponenten und chemischer Struktur der Z-Gruppe. Nach Abkühlung der Reaktionsmischung wird das entstandene Produkt pulverisiert und bei Bedarf aus niedrigen Alkoholen, aliphatischen oder aromatischen Kohlenwasserstoffen durch Rekristallisation gereinigt.

Bei der Reaktion in Lösung erfolgt die Herstellung, indem die Reaktionskomponenten in einem oder mehreren Lösungsmitteln gemischt, aufgeheizt und inerter Atmosphäre bei höheren Temperaturen umgesetzt werden. Lösungsmittel für die Reaktion können aromatische und aprotische hochsiedende Lösungsmittel, bevorzugt Toluen, Xylene, Dimethylformamid, Dimethylsulfoxid, N-methylpyrrolidon, oder halogenierte aromatische Lösungsmittel sein. Die Reaktion in Lösung wird im Temperaturbereich zwischen 100 und 250 °C, vorzugsweise zwischen 140 und 200 °C durchgeführt, wobei die Reaktionszeit zwischen 1 und 12 Stunden, vorzugsweise zwischen 2 und 8 Stunden je nach Reaktionstemperatur, Siedepunkt des Lösungsmittels, Polarität des Lösungsmittels und der chemischen Struktur der Z-Gruppe, beträgt.
Das Reaktionsprodukt wird isoliert, indem es durch Temperaturerniedrigung auf Zimmertemperatur ausgefällt oder indem es nach Verdampfen des Lösungsmittels unter reduziertem Druck rekristallisiert wird. Für die Rekristallisation können niedrige Alkohole, aliphatische oder aromatische Kohlenwasserstoffe verwendet werden. In einigen Fällen kann die Isolierung auch durch Ausfallen der Lösung in Wasser oder anderem Nichtlösungsmittel erfolgen.

Durch das erfindungsgemäße Verfahren können sterisch gehinderte Phenole hergestellt werden, die als Antioxidantien wirken und durch die Komplexierung von im Polymer enthaltenen Katalysatoren ihre stabilisierende Wirkung noch deutlich verbessern.
Eine komplexierende Wirkung wird beispielsweise durch die Gegenwart von zwei eng beieinander liegenden Amidgruppierungen hervorgerufen. Bei dem nach DE 197 47 644 A1 beschriebenen Verfahren sind nur Verbindungen zugänglich, bei denen die Amidgruppierungen zu weit voneinander entfernt sind, um komplexierend wirken zu können.
Da bei dem erfindungsgemäßen Verfahren keine Nebenprodukte entstehen, werden umweltschädigende Wirkungen minimiert und dadurch Herstellungskosten verringert. Ein weiterer Vorteil des Verfahrens besteht darin, dass auf handelsübliche Ausgangsstoffe zurückgegriffen werden kann, was zusätzlichen synthetischen Aufwand verringert.

Diese Erfindung wird weiter durch die mehrere Ausführungsbeispiele näher erläutert.

### Beispiel 1

1,4-Bis(2-oxazolin-2-yl)benzen (0,3 mmol) und 3-(3,5-Di-*tert*-butyl-4-hydroxyphenyl)propionsäure (0,6 mmol) wurden in ein Reagenzglas eingewogen. Das Reagenzglas wurde evakuiert, mehrfach mit Argon gespült und danach verschlossen. Dann wurde das Reagenzglas für eine Stunde auf 180 °C erhitzt. Nach dem Abkühlen wurde ein farbloser glasartiger Feststoff erhalten (0,232 g), welcher in Metanol umkristallisiert wurde. Weiße Kristalle mit einem Schmelzpunkt von 178- 179 ° C wurden erhalten.
Die erfindungsgemäß hergestellten Verbindung schützt Polymere gegen thermooxidativen Abbau und entweicht auch bei höheren Temperaturen nicht aus dem zu stabilisierenden Polymer.
Bei der Synthese werden handelsübliche Ausgangsstoffe verwendet und es entstehen keine Nebenprodukte.

### Beispiel 2

1,4-Bis(2-oxazolin-2-yl)benzen (0,3 mmol) und 3,5-Di-*tert*-butyl-4-hydroxybenzoesäure (0,6 mmol) wurden in ein Reagenzglas eingewogen. Das Reagenzglas wurde evakuiert, mehrfach mit Argon gespült und danach verschlossen. Das Reagenzglas wurde für eine Stunde auf 215°C erhitzt. Nach dem Abkühlen wurde ein farbloser glasartiger Feststoff erhalten (0,215 g), welcher nach längerem Stehen kristallisierte. Der Feststoff wurde in Dimethylformamid (1,5 ml) gelöst und in Wasser (50 ml) ausgefällt. Der weiße Niederschlag wurde abgetrennt, mit Wasser gewaschen und getrocknet. Das erhaltene weiße Pulver (0,200 g ) mit einem Schmelzpunkt von 244-247°C wurde in Metanol umkristallisiert. Weiße Kristalle mit einem Schmelzpunkt von 254 - 256°C wurden erhalten.
Die erfindungsgemäß hergestellten Verbindung schützt Polymere gegen thermooxidativen Abbau und entweicht auch bei höheren Temperaturen nicht aus dem zu stabilisierenden Polymer.
Bei der Synthese werden handelsübliche Ausgangsstoffe verwendet und es entstehen keine Nebenprodukte.

### Beispiel 3

1,4-Bis(2-oxazolin-2-yl)benzen (0,4 mmol) und 3,5-Di-*tert*-butyl-4-hydroxyzimtsäure (0,8 mmol) wurden in ein Reagenzglas eingewogen. Das Reagenzglas wurde evakuiert, mehrere Male mit Argon gespült und verschlossen. Das Reagenzglas wurde für 0,5 Stunden auf 190°C erhitzt. Nach dem Abkühlen wurde ein brauner glasförmiger Feststoff erhalten (0,307 g), welcher in Metanol (3 ml) aufgelöst und in Wasser ausgefällt wurde. Ein gelbliches Pulver wurde abgetrennt mit Wasser gewaschen und getrocknet. Ein gelbliches Pulver (0,265 g ) mit einem Schmelzpunkt von 89 - 92°C wurde erhalten.
Die erfindungsgemäß hergestellten Verbindung schützt Polymere gegen thermooxidativen Abbau und entweicht auch bei höheren Temperaturen nicht aus dem zu stabilisierenden Polymer.
Bei der Synthese werden handelsübliche Ausgangsstoffe verwendet und es entstehen keine Nebenprodukte.

### Beispiel 4

1,3-Bis(2-oxazolin-2-yl)benzen (0,3 mmol) und 3-(3,5-Di-*tert*-butyl-4-hydroxyphenyl)propionsäure (0,6 mmol) wurden in ein Reagenzglas eingewogen, Das Reagenzglas wurde evakuiert, mehrere Male mit Argon gespült und verschlossen. Das Reagenzglas wurde für eine Stunde auf 180 °C erhitzt. Nach dem Abkühlen wurde ein farbloser pulverisierbarer glasartiger Feststoff (0,232 g) mit einem Schmelzpunkt von 59 - 64 °C erhalten.
Die erfindungsgemäß hergestellten Verbindung schützt Polymere gegen thermooxidativen Abbau, entweicht auch bei höheren Temperaturen nicht aus dem zu stabilisierenden Polymer und ist in der Lage, metallische Katalysatoren zu komplexieren.
Bei der Synthese werden handelsübliche Ausgangsstoffe verwendet und es entstehen keine Nebenprodukte.

### Beispiel 5

2,6-Bis(2-oxazolin-2-yl)pyridin (0,2 mmol) und 3-(3,5-Di-*tert*-butyl-4-hydroxyphenyl) propionsäure (0,4 mmol) wurden in ein Reagenzglas eingewogen. Das Reagenzglas wurde evakuiert, mehrere Male mit Argon gespült und verschlossen. Das Reagenzglas wurde für eine Stunde auf 180°C erhitzt. Nach dem Abkühlen wurde ein farbloser glasartiger Feststoff erhalten (0,155 g), welcher in Ethanol umkristallisiert wurde. Weiße Kristalle mit einem Schmelzpunkt von 88 - 91 °C wurden erhalten.
Die erfindungsgemäß hergestellten Verbindung schützt Polymere gegen thermooxidativen Abbau, entweicht auch bei höheren Temperaturen nicht aus dem zu stabilisierenden Polymer und ist in starkem Maße in der Lage, metallische Katalysatoren zu komplexieren.
Bei der Synthese entstehen keine Nebenprodukte.

### Beispiel 6

1,3-Bis(2-oxazolin-2-yl)-5-hydroxybenzen (0,2 mmol) und 3-(3,5-Di-*tert*-butyl-4-hydroxyphenyl)propionsäure (0,4 mmol) wurden in ein Reagenzglas eingewogen. Das Reagenzglas wurde evakuiert, mehrere Male mit Argon gespült und verschlossen. Das Reagenzglas wurde für eine Stunde auf 180 °C erhitzt. Nach dem Abkühlen wurden weiße Kristalle mit einem Schmelzpunkt größer als 360°C erhalten (0,156 g).
Die erfindungsgemäß hergestellten Verbindung schützt Polymere gegen thermooxidativen Abbau, entweicht auch bei höheren Temperaturen nicht aus dem zu stabilisierenden Polymer und ist in der Lage, metallische Katalysatoren zu komplexieren.
Bei der Synthese werden handelsübliche Ausgangsstoffe verwendet und es entstehen keine Nebenprodukte.

### Beispiel 7

1,3-Bis(2-oxazolin-2-yl)-5-allyloxybenzen (0,2 mmol) und 3-(3,5-Di-*tert*-butyl-4-hydroxyphenyl)propionsäure (0,4 mmol) wurden in ein Reagenzglas eingewogen. Das Reagenzglas wurde evakuiert, mehrere Male mit Argon gespült und verschlossen. Das Reagenzglas wurde für eine Stunde auf 180°C erhitzt. Nach dem Abkühlen wurde ein gelblicher pulverisierbarer glasartiger Feststoff mit einem Schmelzpunkt von 59 - 64°C erhalten (0,165 g).
Die erfindungsgemäß hergestellten Verbindung schützt Polymere gegen thermooxidativen Abbau, entweicht auch bei höheren Temperaturen nicht aus dem zu stabilisierenden Polymer und ist in der Lage, metallische Katalysatoren zu komplexieren.
Bei der Synthese werden handelsübliche Ausgangsstoffe verwendet und es entstehen keine Nebenprodukte.

### Beispiel 8

1,4-Bis(2-oxazolin-2-yl)benzen (0,3 mmol) und 3-(3,5-Di-*tert*-butyl-4-hydroxyphenyl)propionsäure (0,6 mmol) und Diphenylformamid (0,5 ml) wurden in ein Reagenzglas gegeben. Das Reagenzglas wurde evakuiert, mehrere Male mit Argon gespült und verschlossen. Das Reagenzglas wurde für sieben Stunde auf 180°C erhitzt. Nach dem Abkühlen wurde die gelbe Lösung in Wasser (25 ml) ausgefällt. Der weiße Niederschlag wurde abgetrennt, mit Wasser gewaschen und getrocknet. Nach der Rekristallisation aus Ethanol wurden weiße Kristalle (0,230 g ) mit einem Schmelzpunkt von 174 - 177°C erhalten .
Die erfindungsgemäß hergestellten Verbindung schützt Polymere gegen thermooxidativen Abbau und entweicht auch bei höheren Temperaturen nicht aus dem zu stabilisierenden Polymer.
Bei der Synthese werden handelsübliche Ausgangsstoffe verwendet und es entstehen keine Nebenprodukte.

### Beispiel 9

1,4-Bis(2-oxazolin-2-yl)benzen (0,3 mmol) und 3-(3,5-Di-*tert*-butyl-4-hydroxyphenyl)propionsäure (0,6 mmol) und Diphenylformamid (0,5 ml) wurden in ein Reagenzglas gegeben. Das Reagenzglas wurde evakuiert, mehrere Male mit Argon gespült und verschlossen. Das Reagenzglas wurde für sieben Stunde auf 180°C erhitzt. Nach dem Abkühlen wurde die gelbe Lösung in Heptan( 20 ml) ausgefällt. Ein weißes Pulver wurde erhalten, welches mit Heptan gewaschen und danach getrocknet wurde. Nach der Rekristallisation aus Ethanol wurden weiße Kristalle (0,217 g) mit einem Schmelzpunkt von 175 - 177°C erhalten .
Die erfindungsgemäß hergestellten Verbindung schützt Polymere gegen thermooxidativen Abbau und entweicht auch bei höheren Temperaturen nicht aus dem zu stabilisierenden Polymer.
Bei der Synthese werden handelsübliche Ausgangsstoffe verwendet und es entstehen keine Nebenprodukte.

### Beispiel 10

1,4-Bis(2-oxazolin-2-yl)benzen (0,3 mmol) und 3-(3,5-Di-*tert*-butyl-4-hydroxyphenyl)propionsäure (0,6 mmol) und Xylene (0,5 ml) wurden in ein Reagenzglas gegeben. Das Reagenzglas wurde evakuiert, mehrere Male mit Argon gespült und verschlossen. Das Reagenzglas wurde für sieben Stunde auf 180°C erhitzt. Nach dem Abkühlen wurden weiße Kristalle separiert und mit Heptan gewaschen und getrocknet. Weiße Kristalle (0,230 g) mit einem Schmelzpunkt von 174 - 177°C wurden erhalten .
Die erfindungsgemäß hergestellten Verbindung schützt Polymere gegen thermooxidativen Abbau und entweicht auch bei höheren Temperaturen nicht aus dem zu stabilisierenden Polymer.
Bei der Synthese werden handelsübliche Ausgangsstoffe verwendet und es entstehen keine Nebenprodukte.

### Beispiel 11

1,4-Bis(2-oxazolin-2-yl)benzen (0,4 mmol) und 3,5-Di-*tert*-butyl-4-hydroxybenzoesäure (0,8 mmol) und Diphenylformamid (0,75 ml) wurden in ein Reagenzglas gegeben. Das Reagenzglas wurde evakuiert, mehrere Male mit Argon gespült und verschlossen. Das Reagenzglas wurde für sieben Stunde auf 180°C erhitzt. Nach dem Abkühlen wurde die Reaktionsmischung in Wasser gefällt. Der weiße Niederschlag wurde separiert, mit Wasser gewaschen und getrocknet. Weiße Kristalle (0,280 g) mit einem Schmelzpunkt von 247 - 250°C wurden erhalten.
Die erfindungsgemäß hergestellten Verbindung schützt Polymere gegen thermooxidativen Abbau und entweicht auch bei höheren Temperaturen nicht aus dem zu stabilisierenden Polymer.
Bei der Synthese werden handelsübliche Ausgangsstoffe verwendet und es entstehen keine Nebenprodukte.

### Beispiel 12

1,4-Bis(2-oxazolin-2-yl)benzen (0,4 mmol) und 3,5-Di-*tert*-butyl-4-hydroxybenzoesäure (0,8 mmol) und Xylen (0,75 ml) wurden in ein Reagenzglas gegeben. Das Reagenzglas wurde evakuiert, mehrere Male mit Argon gespült und verschlossen. Das Reagenzglas wurde für sieben Stunde auf 180°C erhitzt. Nach dem Abkühlen fielen aus der gelben Lösung gelbliche Kristalle aus. Diese wurden separiert, mit Heptan gewaschen und getrocknet. Weiße Kristalle (0,210 g) mit einem Schmelzpunkt von 245 - 249°C wurden erhalten.
Die erfindungsgemäß hergestellten Verbindung schützt Polymere gegen thermooxidativen Abbau und entweicht auch bei höheren Temperaturen nicht aus dem zu stabilisierenden Polymer.
Bei der Synthese werden handelsübliche Ausgangsstoffe verwendet und es entstehen keine Nebenprodukte.

## Patentansprüche

1. Umweltfreundliches Verfahren zur Herstellung sterisch gehinderter Phenole mit höherem Molekulargewicht der allgemeinen Formel (I), 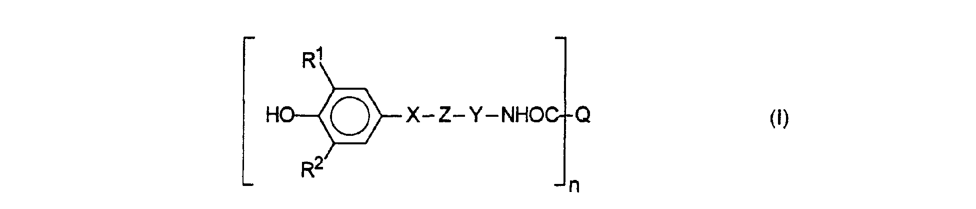
wobei R¹ und R² gleiche oder unterschiedliche, lineare oder verzweigte, monovalente Kohlenwasserstoffgruppen mit 1 bis 8 Kohlenstoffatomen,
X eine einfache chemische Bindung oder eine gesättigte oder ungesättigte Kohlenwasserstoffgruppe mit 1 oder 2 Kohlenstoffatomen,
Y eine Kohlenwasserstoffgruppe mit 2 oder 3 Kohlenstoffatomen,
Z eine -S- oder -COO-Gruppe,
Q ein mono- oder polyvalentes Radikal bestehend aus einer aliphatischen oder zykloalipathischen, gesättigten oder ungesättigten, linearen oder verzweigten Kohlenwasserstoffgruppe mit 1 bis 20 Kohlenstoffatomen, oder
ein mono- oder polyvalentes Radikal bestehend aus einer aliphatischen oder zykloalipathischen, gesättigten oder ungesättigten, linearen oder verzweigten Kohlenwasserstoffgruppe mit 1 bis 20 Kohlenstoffatomen und mindestens einem der Heteroatome S, O, P oder N, oder
ein mono- oder polyvalentes nichtsubstituiertes oder substituiertes aromatisches Radikal bestehend aus 6 bis 20 Kohlenstoffatomen, oder
ein mono- oder polyvalentes nichtsubstituiertes oder substituiertes heteroaromatisches Radikal bestehend aus 5 bis 20 Kohlenstoffatomen, oder
ein mono- oder polyvalentes nichtsubstituiertes oder substituiertes Aralkylradikal bestehend aus 6 bis 20 Kohlenstoffatomen, oder eine Biarylverbindung der allgemeinen Formel (II), 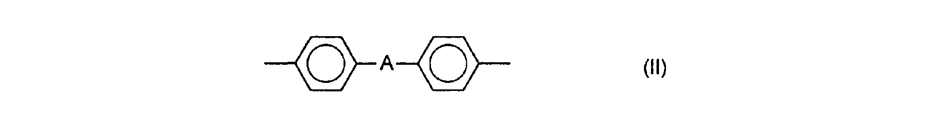
wobei A entweder eine chemische Bindung, eine Methylen-, Isopropyliden-, Isobutyliden-, -S-, -SO-, -SO₂-, -O-, -C(O)-, -OC(O)O- oder -C(O)O-Gruppe repräsentiert, und
n eine ganze Zahl von 1 bis 6, **dadurch gekennzeichnet, dass** eine Verbindung der allgemeinen Formel (III) 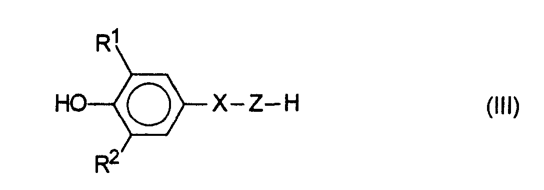 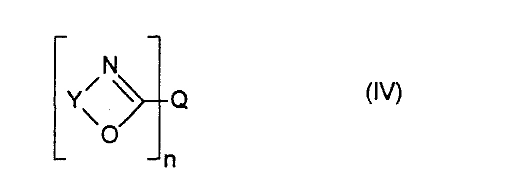
mit einer Verbindung der allgemeinen Formel (IV) zur Reaktion gebracht wird,
wobei in der Verbindung nach Formel (III)
R¹ und R² gleiche oder unterschiedliche lineare oder verzweigte, monovalente Kohlenwasserstoffgruppen mit 1 bis 8 Kohlenstoffatomen,
X eine einfache chemische Bindung oder eine gesättigte oder ungesättigte Kohlenwasserstoffgruppe mit 1 bis 2 Kohlenstoffatomen,
Z eine -S- oder -COO-Gruppe ist, und
in der Verbindung (IV)
Y eine Kohlenwasserstoffgruppe mit 2 oder 3 Kohlenstoffatomen, und
Q ist ein mono- oder polyvalentes Radikal bestehend aus einer aliphatischen, gesättigten oder ungesättigten, linearen oder verzweigten Kohlenstoffgruppe mit 1 bis 20 Kohlenstoffatomen, oder
ein mono- oder polyvalentes Radikal bestehend aus einer aliphatisch oder zykloaliphatischen, gesättigten oder ungesättigten, linearen oder verzweigten Kohlenwasserstoffgruppe mit 1 bis 20 Kohlenstoffatomen und mindestens eins der Heteroatome S, O, P oder N, oder
ein mono- oder polyvalentes nichtsubstituiertes oder substituiertes aromatisches Radikal bestehend aus 6 bis 20 Kohlenstoffatomen, oder
ein mono- oder polyvalentes nichtsubstituiertes oder substituiertes heteroaromatisches Radikal bestehend aus 5 bis 20 Kohlenstoffatomen oder ein mono- oder polyvalentes nichtsubstituiertes oder substituiertes Arylradikal bestehend aus 6 bis 20 Kohlenstoffatomen oder Biarylverbindungen der allgemeinen Formel (II), 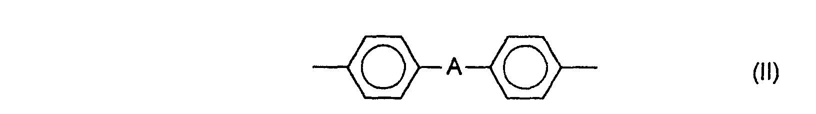
wobei A eine chemische Bindung, eine Methylen-, eine Isopropylen-, eine Isobutylen, eine -S-, -SO-, -SO₂-, -O-, -C(O)-, -OC(O)O- oder -C(O)O-Gruppe repräsentiert, und n eine gerade Zahl von 1 bis 6 ist.

2. Verfahren nach Anspruch 1, **dadurch gekennzeichnet, dass** die Reaktion zwischen den Verbindungen nach Formel (III) und (IV) in Schmelze durchgeführt wird.

3. Verfahren nach Anspruch 2, **dadurch gekennzeichnet, dass** die Reaktion bei einer Reaktionstemperatur in einem Temperaturbereich, der höher als der Schmelzpunkt von mindestens einer der reagierenden Komponenten ist, vorzugsweise zwischen 100 und 260 °C, noch mehr bevorzugt zwischen 150 und 200 °C, durchgeführt wird.

4. Verfahren nach Anspruch 2, **dadurch gekennzeichnet, dass** die Reaktion in einer Zeit zwischen 1 min und 6 h, vorzugsweise zwischen 3 min und 1 h durchgeführt wird.

5. Verfahren nach Anspruch 2, bei dem die Reaktion in Schmelze unter inerter Atmosphäre durchgeführt wird.

6. Verfahren nach Anspruch 1, **dadurch gekennzeichnet, dass** die Reaktion zwischen den Verbindungen nach Formel (III) und (IV) in Lösung durchgeführt wird.

7. Verfahren nach Anspruch 6, **dadurch gekennzeichnet, dass** die Reaktion unter inerter Atmosphäre bei erhöhten Temperaturen durchgeführt wird.

8. Verfahren nach Anspruch 6, **dadurch gekennzeichnet, dass** die Reaktion in einem oder mehreren hochsiedenden Lösungsmitteln, vorzugsweise in aromatischen und aprotischen Lösungsmitteln, wie Toluen, Xylenen, Dimethylformamid, Dimethylsulfoxid, N-Methylpyrrolidon, oder in halogenierten aromatischen Lösungsmitteln durchgeführt wird.

9. Verfahren nach Anspruch 6, **dadurch gekennzeichnet, dass** die Reaktion in einem Temperaturbereich zwischen 100 und 250 °C, bevorzugt zwischen 140 und 200 °C durchgeführt wird und in einer Zeit zwischen 1 und 12 h, vorzugsweise zwischen 2 und 6 h, je nach Reaktionstemperatur, Siedepunkt und Polarität des Lösungsmittels und chemischer Struktur der Z-Gruppe.
